Europäisches Patentamt

European Patent Office

Office européen des brevets

(11) Publication number: **0 383 467**
**A2**

## (12) EUROPEAN PATENT APPLICATION

(21) Application number: 90301191.4

(51) Int. Cl.5: **A61K 7/06**

(22) Date of filing: **05.02.90**

(30) Priority: **06.02.89 ZA 890904**

(43) Date of publication of application:
**22.08.90 Bulletin 90/34**

(84) Designated Contracting States:
**AT BE CH DE DK ES FR GB GR IT LI LU NL SE**

(71) Applicant: **Strydom, Andries Johannes Cornelus**
**21 Dersley Street, Bayswater**
**Bloemfontein, Orange Free State(ZA)**

(72) Inventor: **Strydom, Andries Johannes Cornelus**
**21 Dersley Street, Bayswater**
**Bloemfontein, Orange Free State(ZA)**

(74) Representative: **Baverstock, Michael George Douglas et al**
**BOULT, WADE & TENNANT 27 Furnival Street**
**London, EC4A 1PQ(GB)**

(54) Medicament for use in combatting hair loss and/or dandruff.

(57) A medicament is provided for use in treating hairy or piliferous regions of the body to combat hair loss and/or dandruff. The medicament is applied externally and comprises an admixture of a sugar constituent; a nitrogen-containing constituent; a gum constituent; a terpene and/or a steroid constituent; at least one trace element; a vitamin constituent; and a salt constituent, with the sugar constituent making up approximately 50% by mass, or more, of the admixture.

EP 0 383 467 A2

## MEDICAMENT FOR USE IN COMBATTING HAIR LOSS AND/OR DANDRUFF

THIS INVENTION relates to a medicament. It relates in particular to a medicament for use in treating hairy or piliferous regions of the body to combat hair loss and/or dandruff by external application thereof to the body, and to a method of treating hairy or piliferous regions of the body.

According to a first aspect of the invention, there is provided a medicament for use in treating hairy or piliferous regions of the body to combat hair loss and/or dandruff, by external application thereof to hairy or piliferous regions of the body, the medicament comprising or consisting of an admixture of

a sugar constituent;

a nitrogen-containing constituent;

a gum constituent;

a terpene and/or a steroid constituent;

at least one trace element;

a vitamin constituent; and

a salt constituent,

with the sugar constituent making up approximately 50% by mass, or more, of the admixture.

The sugar constituent may comprise an admixture of monosaccharides, eg glucose, and oligosaccharides, eg sucrose and fructose. The oligosaccharides may make up approximately 50% by mass, or more, of the sugar constituent. The admixture may contain between 50% and 55% by mass, eg approximately 50% by mass, of the sugar constituent.

The nitrogen-containing constituent may make up about 5% by mass, or more, of the admixture. The nitrogen constituent may comprise at least one protein and/or at least one amino-acid, and/or at least one simple nitrogenous compound, such as an amide or an unfermentable sugar, ie a combination of a normal reducing sugar and a nitrogen compound.

The gum constituent may comprise an admixture of gums.

The admixture may contain, as trace element, at least one of cobalt, iron, magnesium, copper and manganese. However, other trace elements may also be present.

The vitamin constituent may include the B-group vitamins, eg $B_6$, as well as choline, inositol, niacin and biotin. It may further include other known vitamins.

The terpene constituent, when present, may comprise at least one of a broad range of known plant terpene compounds and/or a suitable derivative thereof.

The steroid constituent may comprise sterol and/or any other suitable steroid.

The salt constituent may comprise potassium, calcium and phosphorus salts. These salts, when present, may be in a proportion of about 5%, 0.9% and 0.5% respectively, by mass. However, if desired other salts may also be present.

The admixture may, in particular, be molasses, eg crude black molasses, or a refinement thereof such as that available in South Africa from Health-Wise or Nature Food.

The Applicant believes that the medicament can be used to prevent or treat dandruff and/or hair loss by massaging it into the piliferous portions of the body, eg into the scalp.

The medicament may include a compatible liquid carrier, eg water, for the molasses. It may also include a deodorant to mask the smell of molasses. The deodorant may, for example, comprise a simple plant terpene compound having fragrance properties.

The medicament may still further include an adjunct for the molasses. The adjunct may comprise at least one of the following: a sugar, a nitrogen-containing compound, a gum, a terpene constituent, a steroid constituent, a trace element, a vitamin, or a salt. These constituents may be of the same kind as those hereinbefore described.

According to a second aspect of the invention, there is provided a method of treating hairy or piliferous regions of the body to combat hair loss and/or dandruff, which comprises applying externally to hairy or piliferous regions of the body, a medicament according to the first aspect of the invention.

The application may, in particular, be to the scalp of the human body. The application may hence be effected by massaging the medicament into the scalp.

Preferably, the scalp, ie the hair as well as the underlying skin constituting the scalp, are wetted prior to application of the medicament.

The method may hence include washing the hair prior to application of the medicament, and effecting the application of the medicament before the hair dries out. The washing may be effected with an oil removing shampoo.

The application of the medicament may be effected by vigorously rubbing or massaging a suitable quantity, eg 60-80 grams, of the medicament, into the scalp; retaining it on the scalp for a period of time, eg between 1 and 10 minutes, typically about 1 to 2 minutes for crude undiluted molasses; and rinsing it from the scalp with water. The rinsing may be effected repeatedly, but should be done rapidly to minimize rinsing or leaching out of medicament which has penetrated into the scalp.

The application may be effected at least once a day, eg daily, but this can naturally be varied as desired, eg to between 2 and 6 times weekly.

The invention will now be described by way of example, with reference to the following non-limiting example.

A person suffering from hair loss, ie hair which falls out, and dandruff, initially washes his hair with an oil-removing shampoo. The person then rinses his hair and, while it is still damp, vigorously rubs into or massages his scalp with 60-80 grams of undiluted crude black cane molasses, such as that available in South Africa from Health-Wise. The molasses is retained on the scalp for about 1 minute whereafter the scalp is rinsed rapidly three or four times with clean water. The treatment is repeated daily or every second or third day for as long as the condition persists. Hence, to combat hair loss it may be necessary to utilize the treatment on an on-going basis, while for dandruff it can be utilized whenever dandruff is observed. Furthermore, the exuberance of hair re-growth can be modulated by the frequency of application and/or the duration of the treatment and/or by modulating the composition of the medicament such as by dilution or by means of the oily base of a pre-washing shampoo, ie the residual oily compounds remaining on the scalp after use of the pre-washing shampoo.

Dandruff usually arises from a combination of dry skin of the scalp and infection due to different organisms, mainly fungi, although it can also be due to only one of these.

Loss of hair can be ascribed, at least in part, to insufficient supply of nutrients to the hair cells, arising from deteriorating blood circulation, as is often evident in aging people. Certain forms of balding or loss of hair can also be ascribed to male hormones and stress.

These conditions have hitherto been very difficult to overcome. For example, in respect of dandruff it has been found that this can only be curtailed through continuous or on-going use of expensive anti-dandruff shampoos. In the case of hair loss, this has hitherto been thought to be substantially incurable and irreversible in most instances, with limited success only being obtained in some instances with vasodilators. However, it has now surprisingly been found that with the medicament according to the invention, effective control of dandruff and prevention of hair loss, and even hair regrowth, is achieved.

The Applicant further believes that by applying the medicament while the hair is still damp, the medicament is diluted as it is massaged into the scalp, thereby promoting penetration or diffusion thereof into the scalp cells and the hair follicles, which diffusion is further enhanced by pre-washing with a shampoo which removes oil layers from the hair and skin surface.

Once located within or around the cells of the scalp and hair follicles, the medicament is believed to function by providing the necessary nutrients to the surrounding skin and hair cells, thereby to combat degeneration thereof and/or to promote regeneration. The dilution of the molasses as it diffuses through residual water present on the skin surface into the hair cells and follicles is believed to ensure that the medicament is not present in undesirable or harmful concentrations in the hair cells, but rather in a diluted balanced nutritive concentration.

To destroy fungi on the skin surface, the medicament is believed to act directly thereon as a result of the high sugar content of the medicament.

The Applicant has also found that the hair, after treatment in accordance with the method of the invention, can be styled at least to a degree and retain this styling on the hair drying out. This is believed to be due to the gums present in the molasses as well as, possibly, due to components of the molasses, eg sugars, having diffused into the hair fibres. Hence, while a hair conditioner can be used after application of the medicament of the invention, this is believed to be undesirable as it could lead to removal/neutralization of the residual molasses components in or on the hair, with loss of the styling effect and/or the nutrient effect thereof. For this reason it is also believed to be beneficial to rinse the scalp rapidly,eg 3 or 4 times sequentially, rather than once only for a prolonged period.

The Applicant also believes that, since even the protein component of molasses comprises to a large extent small molecules, ie free amino acids and other simple nitrogenous compounds, the molasses will be readily absorbable into the cells of the scalp.

In tests which the Applicant has conducted, it has been found that when molasses is used for treating dandruff in accordance with the invention, dandruff was virtually eliminated by constant and regular usage thereof. It was also found that, when used for combatting hair loss, it inhibits hair loss and even promotes re-growth. Moreover, molasses is cheap and readily available. It is also naturally occurring and safe to use for prolonged periods.

Typically, undiluted crude black cane molasses may comprise, by mass,

| liquid, which is predominantly water - | 20-30%, typically about 25% |
|---|---|
| total sugars - | 48-56%, typically about 50% |
| of which | |
| - sucrose - | 30-40%, typically about 31% |
| - reducing sugars - | 15-20%, typically about 16% |
| - unfermentable sugars - | 2-4%, typically about 3% |
| non-sugar organic matter - | 9-12%, typically about 10% |
| of which | |
| - soluble gums and other carbohydrates - | 3-5%, typically about 4% |
| - organic acids such as aconitic acids - | 2-4%, typically about 3% |
| - organic acids such as citric acid, malic acid, succinic acid, etc - | 0.1-2%, typically about 0.5% |
| - waxes, steroids, terpenes, pigments and vitamins - | 0.1-2%, typically about 0.5% |
| - nitrogen constituents such as protein - | 2-3%, typically about 2% |
| sulphated ash - | 10-15%, typically about 15% |
| of which | |
| - sodium (as Na) - | 0.1-0.4%, typically about 0.3% |
| - potassium (as K) - | 1.5-5.0%, typically about 5.0% |
| - calcium (as Ca) - | 0.4-1.0%, typically about 0.9% |
| - chlorine (as Cl) - | 0.7-3.0%, typically about 2.0% |
| - phosphorus (as P) - | 0.5-2.0%, typically about 0.5% |

## Claims

1. A medicament for use in treating hairy or piliferous regions of the body to combat hair loss and/or dandruff, by external application thereof to hairy or piliferous regions of the body, characterized in that the medicament comprises or consists of an admixture of
a sugar constituent;
a nitrogen-containing constituent;
a gum constituent;
a terpene and/or a steroid constituent;
at least one trace element;
a vitamin constituent; and
a salt constituent,
with the sugar constituent making up approximately 50% by mass, or more, of the admixture.

2. A medicament according to Claim 1, characterized in that the sugar constituent comprises an admixture of monosaccharides and oligosaccharides, with the oligosaccharides making up approximately 50% by mass, or more, of the sugar constituent.

3. A medicament according to Claim 1 or Claim 2, characterized in that the nitrogen-containing constituent makes up about 5% by mass, or more, of the admixture, and comprises at least one protein and/or at least one amino-acid, and/or at least one simple nitrogenous compound.

4. A medicament according to any one of Claims 1 to 3 inclusive, characterized in that the gum constituent comprises an admixture of gums, and wherein the admixture contains, as trace element, at least one of cobalt, iron, magnesium, copper and manganese.

5. A medicament according to any one of Claims 1 to 4 inclusive, characterized in that the vitamin constituent includes the B-group vitamins as well as choline, inositol, niacin and biotin.

6. A medicament according to any one of Claims 1 to 5 inclusive, characterized in that the salt constituent comprises potassium, calcium and phosphorus salts, with these salts being present in the admixture in a proportion of about 5%, 0.9% and 0.5% respectively, by mass.

7. A medicament according to any one of Claims 1 to 6 inclusive, characterized in that the admixture comprises molasses.

8. A medicament according to Claim 7, characterized in that it includes a compatible liquid carrier for the molasses, and a deodorant to mask the smell of molasses.

9. A medicament according to Claim 7 or Claim 8, characterized in that it includes an adjunct for the molasses, the adjunct comprising at least one of the following: a sugar, a nitrogen-containing compound, a gum, a terpene constituent, a steroid constituent, a trace element, a vitamin, or a salt.

10. A method of treating hairy or piliferous regions of the body to combat hair loss and/or dandruff, characterized in that it comprises applying externally to hairy or piliferous regions of the body, a medicament according to any one of Claims 1 to 9 inclusive.

11. A method according to Claim 10, characterized in that it comprises massaging the medicament into the scalp.

12. A method according to Claim 11, characterized in that it includes washing the hair prior to application of the medicament, and effecting the application of the medicament before the hair dries out.

13. A method according to Claim 11 or Claim 12, characterized in that the application of the medicament is effected by vigorously rubbing or massaging 60-80 grams of the medicament, into the scalp; retaining it on the scalp for between 1 and 10 minutes; and rinsing it repeatedly yet rapidly from the scalp with water.

14. A method according to any one of Claims 11 to 13 inclusive, wherein the application is effected at least once a day.

15. The use of molasses in the manufacture of a medicament for treating hairy or piliferous regions of the body to combat hair loss and/or dandruff.

16. The use as claimed in Claim 15 wherein the molasses is mixed with a compatible liquid carrier and a deodorant to mask the smell of molasses.

17. The use as claimed in Claim 15 or Claim 16 wherein the molasses is mixed with an adjunct comprising at least one of the following: a sugar, a nitrogen-containing compound, a gum, a terpene constituent, a steroid constituent, a trace element, a vitamin, or a salt.